# EUROPEAN PATENT APPLICATION

(11) **EP 4 696 423 A1**
(43) Date of publication of application: **18.02.2026**
(21) Application number: 24203057.5
(22) Date of filing: 26.09.2024
(51) Int. Cl.: B06B 1/02, A24F 40/50, A61M 15/00, B05B 17/00

(54) **METHOD AND APPARATUS FOR DRIVING AN ATOMIZING SHEET, ELECTRONIC DEVICE AND STORAGE MEDIUM**

(30) Priority: 12.08.2024 CN 202411108440
(71) Applicant: Feellife Health Inc., Shenzhen, Guangdong 518000 (CN)
(72) Inventor: HUA, Jian, Guangdong, 518000 (CN); ZHENG, Baoda, Guangdong, 518000 (CN)
(74) Representative: Redl, Gerda

(57) **Abstract**

A method and apparatus for driving an atomizing sheet, an electronic device and a storage medium are disclosed, which belong to the technical field of atomizing sheets. The method comprises the following steps of: acquiring atomizing sheet parameters of a target atomizing sheet; acquiring current operating data of the target atomizing sheet; calculating driving parameters of the target atomizing sheet based on the atomizing sheet parameters and the current operating data to obtain a target driving signal; and driving the target atomizing sheet based on the current operating data and the target driving signal. The embodiment of the application is compatible with various types of atomizing sheets, and realizes real-time adjustment according to an operating state of the atomizing sheet, thus improving the control efficiency and accuracy of the atomizing sheet.

## Description

### TECHNICAL FIELD

The present application relates to the technical field of atomizing sheets, and particularly to a method and apparatus for driving an atomizing sheet, an electronic device and a storage medium.

### BACKGROUND

An atomizing sheet is often used to transform a liquid substance into tiny atomized particles, which is widely used in a medical atomizer and other instruments, and can effectively deliver a drug to a part to be treated.

However, current circuits for driving atomizing sheets are not compatible with various types of atomizing sheets, leading to the need of providing special driving circuits for different atomizing sheets for control in practical application, so that it is difficult to monitor and adjust the driving states of different types of atomizing sheets in real time, thus reducing the overall control efficiency.

Therefore, how to improve the control efficiency of the atomizing sheet has become an urgent technical problem.

### SUMMARY

An embodiment of the present application is mainly intended to provide a method and apparatus for driving an atomizing sheet, an electronic device and a storage medium, aiming at improving the control efficiency of an atomizing sheet.

In a first aspect of the present application, an embodiment provides a method for driving an atomizing sheet, including:
acquiring atomizing sheet parameters of a target atomizing sheet;
acquiring current operating data of the target atomizing sheet;
calculating driving parameters of the target atomizing sheet based on the atomizing sheet parameters and the current operating data to obtain a target driving signal; and
driving the target atomizing sheet based on the current operating data and the target driving signal.

In some embodiments, the calculating the driving parameters of the target atomizing sheet based on the atomizing sheet parameters and the current operating data to obtain the target driving signal includes:
determining reference operating data according to the atomizing sheet parameters;
performing frequency sweeping on the target atomizing sheet based on the reference operating data and the current operating data to obtain an original driving frequency; and
generating the target driving signal based on the original driving frequency.

In some embodiments, the current operating data includes a current operating frequency and a current operating current, the reference operating data includes a target operating frequency range, and the performing frequency sweeping on the target atomizing sheet based on the reference operating data and the current operating data to obtain the original driving frequency includes:
acquiring a maximum value of the current operating current in a preset frequency sweeping period to obtain a maximum operating current;
determining a maximum current operating moment according to the maximum operating current;
acquiring a maximum current operating frequency from the current operating frequency based on the maximum current operating moment; and
in response to the maximum current operating frequency being within the target operating frequency range, determining the maximum current operating frequency as the original driving frequency.

In some embodiments, the generating the target driving signal based on the original driving frequency includes:
acquiring an original driving amplitude from the current operating data according to the original driving frequency; and
modulating a driving signal based on the original driving frequency and the original driving amplitude to obtain the target driving signal.

In some embodiments, the current operating data further includes current operating power and a current operating temperature, the reference operating data includes preset minimum operating power and a preset low-temperature operating threshold, and the performing frequency sweeping on the target atomizing sheet based on the reference operating data and the current operating data to obtain the original driving frequency includes:
in response to the current operating power being lower than the minimum operating power or the current operating temperature is lower than the low-temperature operating threshold, carrying out the performing frequency sweeping on the target atomizing sheet based on a preset number of times of frequency sweeping to obtain a plurality of initial driving frequencies; and
averaging the plurality of initial driving frequencies to obtain the original driving frequency.

In some embodiments, the reference operating data further includes a preset hole plugging operating power range, and the driving the target atomizing sheet based on the current operating data and the target driving signal includes:
in response to the current operating power being within the hole plugging operating power range, performing frequency conversion on the target driving signal based on the reference operating data to obtain a variable frequency driving signal; and
driving the target atomizing sheet based on the variable frequency driving signal.

In some embodiments, the reference operating data further includes a preset target operating power range, and after driving the target atomizing sheet based on the variable frequency driving signal, the method further includes:
acquiring variable frequency operating power of the target atomizing sheet; and
in response to the variable frequency operating power being within the target operating power range, driving the target atomizing sheet based on the target driving signal.

In a second aspect of the present application, an embodiment provides an apparatus for driving an atomizing sheet, where the apparatus includes:
an atomizing sheet parameter acquisition module configured for acquiring atomizing sheet parameters of a target atomizing sheet;
a current operating data acquisition module configured for acquiring current operating data of the target atomizing sheet;
a driving parameter calculation module configured for calculating driving parameters of the target atomizing sheet based on the atomizing sheet parameters and the current operating data to obtain a target driving signal; and
an atomizing sheet driving module configured for driving the target atomizing sheet based on the current operating data and the target driving signal.

In a third aspect of the present application, an embodiment provides an electronic device, where the electronic device includes a memory and a processor, the memory stores a computer program, and when executing the computer program, the processor implements the method in the first aspect above.

In a fourth aspect of the present application, an embodiment provides a computer-readable storage medium, where the computer-readable storage medium stores a computer program, when executed by a processor, causes the processor to perform the method in the first aspect above.

The method and apparatus for driving an atomizing sheet, the electronic device and the storage medium, provided by the present application, provides compatibility with various types of atomizing sheets, and real-time adjustment according to an operating state of the atomizing sheet, which improves the control efficiency and accuracy of the atomizing sheet. This is achieved by the electronic device and storage medium acquiring the atomizing sheet parameters of the target atomizing sheet, acquiring the current operating data of the target atomizing sheet, calculating the driving parameters of the target atomizing sheet based on the atomizing sheet parameters and the current operating data to obtain the target driving signal, and driving the target atomizing sheet based on the current operating data and the target driving signal.

### BRIEF DESCRIPTION OF THE DRAWINGS

FIG. 1 is a flow chart of a method for driving an atomizing sheet provided by an embodiment of the present application;
FIG. 2 is a flow chart of step S103 in FIG. 1;
FIG. 3 is a flow chart of step S202 in FIG. 2;
FIG. 4 is another flow chart of step S202 in FIG. 2;
FIG. 5 is a flow chart of step S203 in FIG. 2;
FIG. 6 is a flow chart of step S104 in FIG. 1;
FIG. 7 is a flow chart of a method for driving an atomizing sheet provided by another embodiment of the present application;
FIG. 8 is a schematic structural diagram of an apparatus for driving an atomizing sheet provided by the embodiment of the present application; and
FIG. 9 is a schematic structural diagram of hardware of an electronic device provided by the embodiment of the present application.

### DETAILED DESCRIPTION

To make the objects, the technical solutions, and the advantages of the present application clearer, the present application is further described in detail hereinafter with reference to the drawings and embodiments. It should be understood that the specific embodiments described herein are only used for explaining the present application and are not intended to limit the present application.

It should be noted that although the functional module division is performed in the schematic diagram of the apparatus and the logical sequence is shown in the flow chart, the steps shown or described can be executed by the module division different from that in the apparatus or the sequence different from that in the flow chart in some cases. The terms "first", "second", etc. in the specification, the claims, and the drawings above are used to distinguish similar objects, and are not necessarily used to describe a specific order or sequence.

Unless otherwise defined, all technical and scientific terms used herein have the same meanings as those commonly understood by those skilled in the technical field of the present application. The terms used herein are only for the purpose of describing the embodiments of the present application, and are not intended to limit the present application.

An atomizing sheet is a component used to transform a liquid drug or other solutions into tiny atomized particles, which is usually made of a special material, and can vibrate and atomize a liquid rapidly by an ultrasonic wave, an airflow, or other methods, so as to form inhalable fine particles to facilitate inhalation by patients, thus achieving the direct action of the drug in respiratory tract or lungs, and having the effects of quick response, high efficiency, and the like in treating respiratory diseases such as asthma and chronic obstructive pulmonary disease (COPD).

However, with the increase of types of atomizing sheets, current circuits for driving atomizing sheet are not compatible with various types of atomizing sheets, leading to the need of providing special driving circuits for different atomizing sheets for control in practical application, so that it is difficult to monitor and adjust driving states of different types of atomizing sheets in real time, thus reducing the overall control efficiency.

On this basis, an embodiment of the present application provides a method and apparatus for driving an atomizing sheet, an electronic device and a storage medium, aiming at improving the control efficiency of an atomizing sheet.

The method and apparatus for driving an atomizing sheet, the electronic device and the storage medium provided by the embodiment of the present application are described in detail hereinafter with reference to the following embodiments, and the method for driving an atomizing sheet in the embodiment of the present application will be described first.

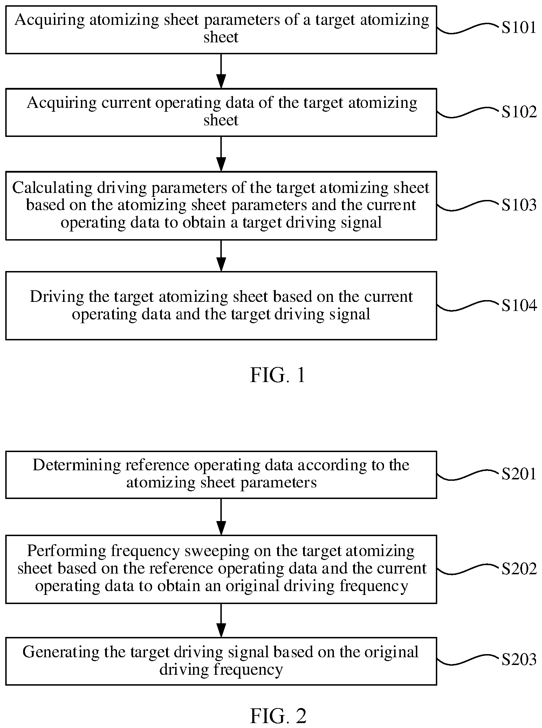

FIG. 1 is an optional flow chart of the method for driving atomizing sheet provided by the embodiment of the present application, and the method in FIG. 1 includes, but is not limited to, step S101 to step S104.

In step S101, atomizing sheet parameters of a target atomizing sheet are acquired.

In step S102, current operating data of the target atomizing sheet is acquired.

In step S103, driving parameters of the target atomizing sheet are calculated based on the atomizing sheet parameters and the current operating data to obtain a target driving signal.

In step S104, the target atomizing sheet is driven based on the current operating data and the target driving signal.

According to step S 101 to step S104 shown in the embodiment of the present application, by acquiring the atomizing sheet parameters of the target atomizing sheet; acquiring the current operating data of the target atomizing sheet; calculating the driving parameters of the target atomizing sheet based on the atomizing sheet parameters and the current operating data to obtain the target driving signal; and driving the target atomizing sheet based on the current operating data and the target driving signal, the compatibility with various types of atomizing sheets is realized, and real-time adjustment can be achieved according to an operating state of each atomizing sheet, which improves the control efficiency and accuracy of the atomizing sheet.

In addition, the method for driving an atomizing sheet provided by the embodiment of the present disclosure is compatible with various types of atomizing sheets, without needing to separately arrange special driving circuits for different atomizing sheets for control, thus reducing the use cost of the atomizing sheet, and improving the use experience and convenience.

In step S 101 of some embodiments, the atomizing sheet parameters of the target atomizing sheet can be corresponding atomizing sheet parameters of the target atomizing sheet that are obtained through Wi-Fi, Bluetooth, RF, and other methods, and input by a technician/user; or can be corresponding atomizing sheet parameters of the target atomizing sheet that are input by the technician/user through a button, a switch, and other methods of a control panel/display screen.

Specifically, the atomizing sheet parameters include, but are not limited to, an atomizing sheet type, an atomizing sheet material, an atomizing sheet size, a rated operating voltage and a rated operating current of the target atomizing sheet.

The atomizing sheet type includes a solid-hole atomizing sheet, a micro-hole atomizing sheet, a mesh atomizing sheet, and the like. The atomizing sheet material includes a ceramic material, a metal material (Pd-Ni alloy, steel metal and titanium-plated metal), a plastic (PI, PP, PE, PET, etc.), and the like.

In step S 102 of some embodiments, the current operating data includes a current operating frequency, a current operating amplitude, a current operating current, current operating power and a current operating temperature.

The current operating frequency is used to represent a number of times of vibration of the atomizing sheet per second, and can be used to measure a working speed and capacity of the atomizing sheet. Generally speaking, the higher the frequency, the smaller the particles produced by the atomizing sheet, thus forming a finer atomized substance.

The current operating amplitude is used to represent a vibration displacement or a vibration range of the atomizing sheet during working. Specifically, the current operating amplitude reflects a maximum distance that a surface or specific area of the atomizing sheet deviates from a static position during high-frequency vibration.

It should be noted that, when the target atomizing sheet starts to work, the current operating frequency and the current operating amplitude can be identified according to the driving signal of the target atomizing sheet, and a specific identification method can be selected according to an actual application scenario, which is not limited to this.

It can be understood that, according to a power calculation formula P=UI, where P is power, U is a voltage and I is a current, because the atomizing sheet is usually operated according to the rated operating voltage during operating, the current operating power can be equal to the rated operating voltage multiplied by the current operating current, that is:
current operating power = rated operating voltage * current operating current.

With reference to FIG. 2, in some embodiments, step S103 includes, but is not limited to, step S201 to step S203.

In step S201, reference operating data is determined according to the atomizing sheet parameters.

In step S202, frequency sweeping is performed on the target atomizing sheet based on the reference operating data and the current operating data to obtain an original driving frequency.

In step S203, the target driving signal is generated based on the original driving frequency.

According to step S201 to step S203 illustrated in the embodiment of the present application, by determining the reference operating data according to the specific parameters of the atomizing sheet and performing frequency sweeping on the target atomizing sheet in combination with the current operating data, the original driving frequency most suitable for a current working condition can be accurately identified. By generating the target driving signal based on the original driving frequency, it can be ensured that the atomizing sheet is operated in the best state, thus improving the atomizing efficiency of the atomizing sheet, and effectively avoiding the performance degradation or resource waste caused by frequency mismatch.

In some embodiments, the reference operating data includes, but is not limited to, a target operating frequency range, preset minimum operating power, a preset low-temperature operating threshold, a preset hole plugging operating power range and a preset target operating power range.

In some embodiments, the target operating frequency range of the target atomizing sheet can be determined according to the atomizing sheet type, the atomizing sheet material and the atomizing sheet size.

For example:
in the case of the target atomizing sheet type being the micro-hole atomizing sheet, the atomizing sheet material is a PI material, and the atomizing sheet size is 13 mm in diameter, the target operating frequency range of the target atomizing sheet is 110 kHz to 130 kHz, which is converted into a mathematical interval [110, 130], in a unit of kHz; and
in the case of the target atomizing sheet type being the micro-hole atomizing sheet, the atomizing sheet material is a stainless steel material, and the atomizing sheet size is 16 mm in diameter, the target operating frequency range of the target atomizing sheet is 2.0 MHz to 2.5 MHz, which is converted into a mathematical interval [2.0, 2.5], in a unit of MHz.

It should be noted that the target operating frequency range of the target atomizing sheet is a preset value, and a specific value needs to be determined according to physical characteristics of the atomizing sheet material, an atomizing liquid material and an atomizer design, which is not limited to this.

In some embodiments, the operating power threshold of the target atomizing sheet can be determined according to the atomizing sheet type, the rated operating voltage and the rated operating current, where the operating power threshold includes minimum operating power P1, normal operating power P2, maximum allowable operating power P3 and dangerous operating power P4.

Specifically, a relationship among the minimum operating power P 1, the normal operating power P2, the maximum allowable operating power P3 and the dangerous operating power P4 is: P1< P2< P4< P3.

The preset minimum operating power corresponds to the minimum operating power P1.

The preset hole plugging operating power range is P4 to P3, which is converted into a mathematical interval (P4, P3], in a unit of watts; and
the preset target operating power range is P1 to P2, which is converted into a mathematical interval (P 1, P2], in a unit of watts.

It should be noted that the operating power threshold of the target atomizing sheet needs to be determined according to the physical characteristics of the atomizing sheet material, and a rated operating voltage and rated operating current of the atomizer, which is not limited to this.

In some embodiments, the preset low-temperature operating threshold of the target atomizing sheet can be determined according to the atomizing sheet type and the atomizing sheet material.

It can be understood that the method for driving an atomizing sheet provided by the embodiment of the present application needs to be combined with the atomizing sheet parameters when generating the target driving signal.

For example:
if the target atomizing sheet is the stainless steel atomizing sheet, and input atomizing sheet parameters are the parameters of the PI atomizing sheet, then the target driving signal is a corresponding driving signal of the PI atomizing sheet; and
if the target atomizing sheet is the PI atomizing sheet, and input atomizing sheet parameters are the parameters of the PI atomizing sheet, then the target driving signal is a corresponding driving signal of the target atomizing sheet (PI atomizing sheet).

By combining with the atomizing sheet parameters to generate the target driving signal, the technician/user is provided with a more freedom adjustment space, so that the technician/user can drive according to different types of atomizing sheets, thus realizing the compatibility with various types of atomizing sheets, and improving the control efficiency, accuracy and freedom of the atomizing sheet.

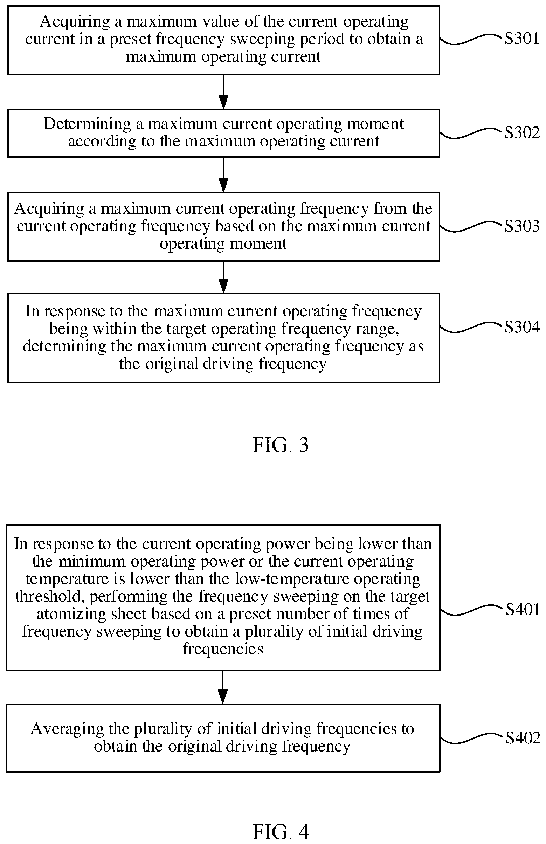

With reference to FIG. 3, in some embodiments, step S202 includes, but is not limited to, step S301 to step S304.

In step S301, a maximum value of the current operating current in a preset frequency sweeping period is acquired to obtain a maximum operating current.

In step S302, a maximum current operating moment is determined according to the maximum operating current.

In step S303, a maximum current operating frequency is acquired from the current operating frequency based on the maximum current operating moment.

In step S304, in response to the maximum current operating frequency being within the target operating frequency range, the maximum current operating frequency is determined as the original driving frequency.

According to step S301 to step S304 illustrated in the embodiment of the present application, by monitoring the maximum value of the current operating current in the preset frequency sweeping period, and determining the maximum current operating moment according to the maximum operating current, a corresponding maximum current operating frequency is extracted from the current operating frequency, so that dynamic response characteristics of the target atomizing sheet are accurately captured. When it is determined that the maximum current operating frequency falls within the preset target operating frequency range, the maximum current operating frequency is directly determined as the original driving frequency, which simplifies the complexity of frequency tuning, ensures the accuracy and efficiency of the driving signal, and improves the compatibility with different types of atomizing sheets.

It should be noted that the current operating frequency and the current operating amplitude can form a current amplitude-frequency characteristic curve of the target atomizing sheet.

It can be understood that, when the driving signal is mismatched, the operating power of the atomizing sheet is unstable, and according to characteristics of the atomizing sheet, at the moment when the operating power reaches the maximum, a corresponding frequency point in the current amplitude-frequency characteristic curve is the optimal operating frequency.

Moreover, the atomizing sheet is usually operated according to the rated operating voltage, and according to a power calculation formula P=UI, the power is the maximum when the current is the maximum. Therefore, in the preset frequency sweeping period, the maximum operating current is obtained by acquiring the maximum value of the current operating current, and a maximum current operating moment is acquired, so that the maximum current operating frequency can be acquired from the current operating frequency according to the maximum current operating moment.

Finally, the maximum current operating frequency is compared with the target operating frequency range, and when the maximum current operating frequency is within the target operating frequency range, the maximum current operating frequency is determined as the original driving frequency.

For example, when the target atomizing sheet type is the micro-hole atomizing sheet, the atomizing sheet material is a PI material, and the atomizing sheet size is 13 mm in diameter, the target operating frequency range of the target atomizing sheet is 110 kHz to 130 kHz.

The maximum current operating frequency obtained by frequency sweeping is 120 kHz, and after comparison, it is found that 120 kHz is within the target operating frequency range of 110 kHz to 130 kHz, so that the original driving frequency can be determined to be 120 kHz.

In some embodiments, step S304 includes, but is not limited to, the following steps.

In response to the maximum current operating frequency being within the target operating frequency range, and the maximum operating power is within the target operating power range, the maximum current operating frequency is determined as the original driving frequency; where the maximum operating power is calculated according to the rated operating power and the maximum operating current.

Specifically, the maximum operating power is equal to the rated operating voltage multiplied by the maximum operating current.

The maximum operating power within the target operating power range is: minimum operating power P1 < maximum operating power < normal operating power P2.

According to step S202 of some embodiments, based on a frequency recorded in the target operating frequency range, it is also possible to gradually sweep the frequency from a lower frequency limit to an upper frequency limit to find the optimal driving frequency, the operating power of the target atomizing sheet is within the target operating power range at the optimal driving frequency, and the optimal driving frequency is taken as the original driving frequency.

For example, when the target atomizing sheet type is the micro-hole atomizing sheet, the atomizing sheet material is a PI material, and the atomizing sheet size is 13 mm in diameter, the target operating frequency range of the target atomizing sheet is 110 kHz to 130 kHz.

Then, starting from the lower frequency limit of 110 kHz, the frequency is gradually swept to the upper frequency limit of 130 kHz to find the optimal driving frequency (such as 120 kHz), and the original driving frequency is determined to be 120 kHz.

It should be noted that, due to physical characteristics of the atomizing sheet, in response to the current operating power being lower than the minimum operating power or the current operating temperature is lower than the low-temperature operating threshold, the original driving frequency obtained by one-time frequency sweeping is inaccurate, so that repeated frequency sweeping needs to be performed on the target atomizing sheet, which is specifically described by the following embodiments.

With reference to FIG. 4, in some embodiments, step S202 includes, but is not limited to, step S401 to step S402.

In step S401, in response to the current operating power being lower than the minimum operating power or the current operating temperature is lower than the low-temperature operating threshold, the frequency sweeping is performed on the target atomizing sheet based on a preset number of times of frequency sweeping to obtain a plurality of initial driving frequencies.

In step S402, the plurality of initial driving frequencies are averaged to obtain the original driving frequency.

According to step S401 to step S402 of the embodiment of the present application, in response to the current operating power being lower than the minimum operating power or the current operating temperature is lower than the low-temperature operating threshold, the frequency sweeping is performed on the target atomizing sheet for many times based on the preset number of times of frequency sweeping to obtain the plurality of initial driving frequencies, and the original driving frequency is obtained by average calculation, which can smooth a possible local fluctuation, ensure the stability of the obtained original driving frequency, and avoid operating instability caused by an extreme working condition.

It should be noted that the specific embodiment of performing frequency sweeping on the target atomizing sheet based on the preset number of times of frequency sweeping to obtain the plurality of initial driving frequencies is basically the same as the specific embodiment of step S301 to step S303 above, which will not be repeated herein, except that the finally obtained maximum current operating frequency is determined as the original driving frequency.

It should be noted that the preset number of times of frequency sweeping can be five, eight or ten, which needs to be set according to the atomizing sheet type, the atomizing sheet material and an actual application scene of the atomizing sheet, which is not limited to this.

In some embodiments, the plurality of initial driving frequencies are averaged, and it is also necessary to judge whether the obtained original driving frequency is within the target operating frequency range.

For example, when the target atomizing sheet type is the micro-hole atomizing sheet, the atomizing sheet material is a PI material, and the atomizing sheet size is 13 mm in diameter, the target operating frequency range of the target atomizing sheet is 110 kHz to 130 kHz.

The frequency sweeping is repeatedly performed on the target atomizing sheet for five times to obtain five initial driving frequencies, which are namely 105 kHz, 110 kHz, 115 kHz, 115 kHz and 125 kHz.

The five initial driving frequencies are averaged to obtain an original driving frequency of 114 kHz, and 114 kHz is within the target operating frequency range of 110 kHz to 130 kHz.

In some embodiments, step S402 includes, but is not limited to, the following steps.

In response to the frequency sweeping being performed on the target atomizing sheet for the last time, the last initial driving frequency is within the target operating frequency range, or the maximum operating power is within the target operating power range, and the plurality of initial driving frequencies are averaged to obtain the original driving frequency.

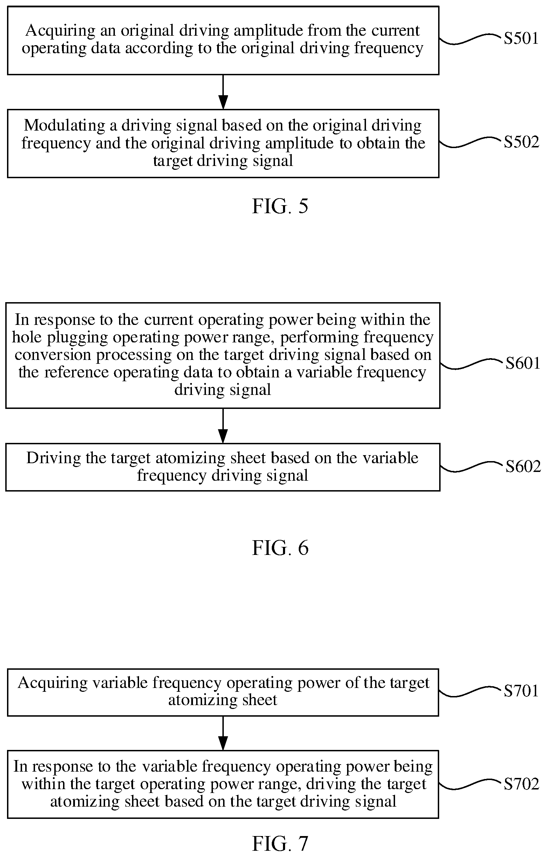

With reference to FIG. 5, in some embodiments, step S203 further includes, but is not limited to, step S501 to step S502.

In step S501, an original driving amplitude is acquired from the current operating data according to the original driving frequency.

In step S502, a driving signal is modulated based on the original driving frequency and the original driving amplitude to obtain the target driving signal.

According to step S501 to step S502 illustrated by the embodiment of the present application, by accurately extracting the original driving amplitude from the current operating data according to the original driving frequency, and combining the original driving frequency and the original driving amplitude to modulate the driving signal, the target driving signal is generated, and actual response characteristics of the atomizing sheet in a current state are fully utilized, which ensures accurate matching between the driving signal and an atomizing sheet performance, reduces unnecessary energy consumption and potential vibration noise, and improving the control efficiency of the atomizing sheet.

It can be understood that, when the original driving frequency is determined, that is, when the maximum current operating moment is determined, an amplitude value in the current amplitude-frequency characteristic curve at this moment can be acquired to obtain the original driving amplitude.

Further, the driving signal can be modulated based on the original driving frequency and the original driving amplitude to obtain the target driving signal.

The target driving signal can be used to change the operating frequency and the operating amplitude of the target atomizing sheet, which can be specifically shown in the amplitude-frequency characteristic curve.

In some embodiments, the reference operating data further includes a preset frequency conversion rule; where the frequency conversion rule needs to be set according to the atomizing sheet type, the atomizing sheet material and the actual application scene of the atomizing sheet, which is not limited to this.

With reference to FIG. 6, in some embodiments, step S104 includes, but is not limited to, step S601 to step S602.

In step S601, in response to the current operating power being within the hole plugging operating power range, frequency conversion processing is performed on the target driving signal based on the reference operating data to obtain a variable frequency driving signal.

In step S602, the target atomizing sheet is driven based on the variable frequency driving signal.

According to step S601 to step S602 illustrated in the embodiment of the present application, by judging whether the current operating power is within the hole plugging operating power range, in response to the current operating power being within the hole plugging operating power range, it is indicated that the operating power of the target atomizing sheet can increase abnormally due to a plugged air hole in the atomizing sheet. Therefore, the frequency conversion processing needs to be performed on the target driving signal based on the reference operating data to generate the variable frequency driving signal, and by dynamically adjusting the frequency of the driving signal, abnormal situations such as hole plugging can be effectively handled, and possible damage or atomization efficiency reduction caused by excessively large power can be reduced.

In addition, the perforation of the atomizing sheet caused by excessively large operating power can also be avoided, thus avoiding the potential safety hazard caused by the inhalation of atomizing sheet fragments into lungs by a user.

In step S601 of some embodiments, in response to the current operating power being within the hole plugging operating power range, that is, when the dangerous operating power P4 < the current operating power ≤ the maximum allowable operating power P3, it is judged that the target atomizing sheet is in a hole plugging operating state, and the frequency conversion processing is performed on the target driving signal according to the frequency conversion rule to obtain the variable frequency driving signal.

For example:
the target driving signal includes the original driving frequency and the original driving amplitude, where the original driving frequency is recorded as T and the original driving amplitude is recorded as H, and the frequency conversion rule is as follows:
in a first time period, the original driving frequency is adjusted to be T+N, and the original driving amplitude is adjusted to be H+M;
in a second time period, the original driving frequency is adjusted to be T+N, and the original driving amplitude is adjusted to be H+2M; and
in a third path time period, the original driving frequency is adjusted to be T-N, and the original driving amplitude is adjusted to be H-M.

In each time period, the driving signal is modulated to obtain a corresponding variable frequency driving signal of this time period, so as to drive the target atomizing sheet in real time based on the variable frequency driving signal.

The first time period, the second time period and the third time period form one frequency conversion cycle.

It should be noted that N and M in each time period can be the same or different, which needs to be set according to the actual application scenario, and is not limited to this.

In addition, how many frequency conversion cycles does the frequency conversion processing need to complete specifically is determined according to the frequency conversion rule, and the frequency conversion rule needs to be set according to the atomizing sheet type, the atomizing sheet material and the actual application scenario of the atomizing sheet.

With reference to FIG. 7, in some embodiments, after step S602, the method for driving atomizing sheet includes, but is not limited to, step S701 to step S702.

In step S701, variable frequency operating power of the target atomizing sheet is acquired.

In step S702, in response to the variable frequency operating power being within the target operating power range, the target atomizing sheet is driven based on the target driving signal.

According to step S701 to step S702 illustrated in the embodiment of the present application, when it is judged that the target atomizing sheet is in the hole plugging operating state, the target atomizing sheet can be subjected to frequency conversion control to achieve a hole plugging prevention effect. The variable frequency operating power of the target atomizing sheet can be monitored and acquired in real time in the process of frequency conversion control. Once the variable frequency operating power is determined to be within the target operating power range, the frequency conversion control is withdrawn, and the target atomizing sheet is driven based on the target driving signal. The operating state of the target atomizing sheet can be dynamically adjusted and finely regulated to ensure the long-term stable operation of the target atomizing sheet, thus improving the control efficiency of the atomizing sheet.

The target driving signal is obtained by the specific embodiment illustrated in step S201 to step S203 above, which will not be repeated herein.

In some embodiments, if the current operating power exceeds the maximum allowable operating power P3, the target atomizing sheet is in a dry burning and heating state, the driving of the target atomizing sheet is suspended, and a prompt message is sent to remind the technician/user to check.

The method for driving an atomizing sheet provided by the embodiment of the present application is compatible with various types of atomizing sheets, can realize real-time adjustment according to the operating state of the atomizing sheet, and has a strong anti-interference ability, thus ensuring the stability of the driving signal.

In addition, when the driving signal is calculated for a specific atomizing sheet, the driving signal can be calculated in combination with the input atomizing sheet parameters, so that the technician/user can perform customized driving according to different types of atomizing sheets, and provide supports for various types of atomizing sheets, thus improving the control efficiency, accuracy and freedom of the atomizing sheet.

In addition, according to the method for driving an atomizing sheet provided by the embodiment of the present application, whether the atomizing sheet is in the hole plugging operating state can also be judged, and when the atomizing sheet is in the hole plugging operating state, the frequency of the driving signal can be dynamically adjusted through the frequency conversion processing, thus effectively dealing with the abnormal situation of hole plugging, and reducing the occurrence of hole plugging.

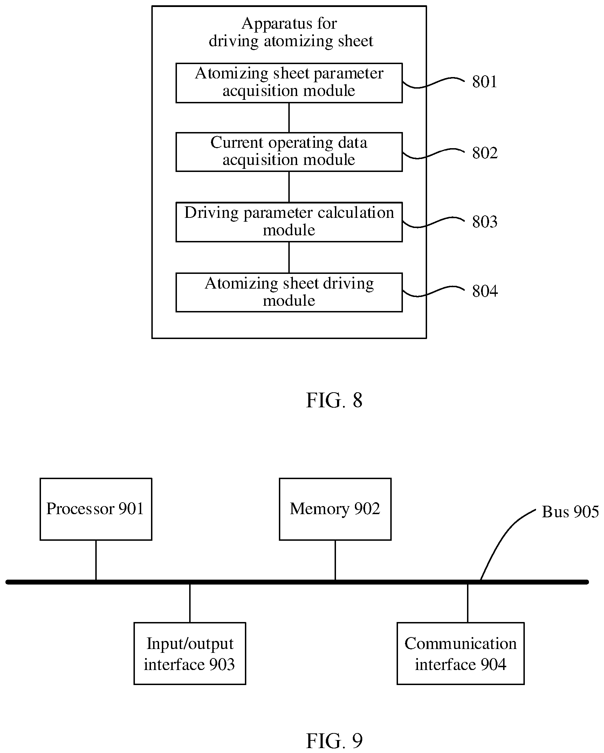

With reference to FIG. 8, the embodiment of the present application further provides an apparatus for driving an atomizing sheet, capable of implementing the method for an driving atomizing sheet above, where the apparatus includes:
an atomizing sheet parameter acquisition module 801 configured for acquiring atomizing sheet parameters of a target atomizing sheet;
a current operating data acquisition module 802 configured for acquiring current operating data of the target atomizing sheet;
a driving parameter calculation module 803 configured for calculating driving parameters of the target atomizing sheet based on the atomizing sheet parameters and the current operating data to obtain a target driving signal; and
an atomizing sheet driving module 804 configured for driving the target atomizing sheet based on the current operating data and the target driving signal.

The specific embodiment of the apparatus for driving an atomizing sheet is basically the same as the specific embodiment of the atomizing blade driving method described above, which will not be repeated herein.

The embodiment of the present application further provides an electronic device, where the electronic device includes a memory and a processor, the memory stores a computer program, and when executing the computer program, the processor implements the method for driving an atomizing sheet above. The electronic device can be any intelligent terminal including a tablet computer, a vehicle-mounted computer, and the like.

With reference to FIG. 9, FIG. 9 shows a structure of hardware of an electronic device of another embodiment, and the electronic device includes:
a processor 901, which can be realized by a general CPU (Central Processing Unit), microprocessor, and Application Specific Integrated Circuit (ASIC), or one or more integrated circuits, and is used for executing related programs to realize the technical solution provided by the embodiment of the present application;
a memory 902, which can be realized in a form of Read Only Memory (ROM), static storage device, dynamic storage device or Random Access Memory (RAM), where the memory 902 can store an operating system and other application programs, and when the technical solution provided by the embodiment in the specification is realized by software or firmware, relevant program codes are stored in the memory 902, and called by the processor 901 to execute the method for driving an atomizing sheet of the embodiment of the present application;
an input/output interface 903, which is used for realizing information input and output;
a communication interface 904, which is used for realizing communication interaction between the device and other devices, where the communication can be realized by a wired method (such as a USB, a network cable, etc.) or a wireless method (such as a mobile network, WIFI, Bluetooth, etc.); and
a bus 905, which is used for transmitting information among various components of the device (such as the processor 901, the memory 902, the input/output interface 903 and the communication interface 904).

The processor 901, the memory 902, the input/output interface 903 and the communication interface 904 communicate with each other inside the device through the bus 905.

The embodiment of the present application further provides a computer-readable storage medium, where the computer-readable storage medium stores a computer program, when executed by a processor, causes the processor to perform the method for driving an atomizing sheet above.

The memory is used as a non-transient computer-readable storage medium, and can be used for storing a non-transient software program and a non-transient computer-executable program. In addition, the memory can include a high-speed random access memory, and can further include a non-transient memory, such as at least one disk memory device, flash memory device, or other non-transient solid-state memory devices. In some embodiments, the memory can optionally include a memory remotely arranged relative to the processor, and these remote memories can be connected to the processor through a network. Examples of the network above include, but are not limited to, the Internet, the Intranet, a local region network, a mobile communication network, and a combination thereof.

According to the method and apparatus for driving an atomizing sheet, the electronic device and the storage medium provided by the embodiment of the present application, by acquiring the atomizing sheet parameters of the target atomizing sheet; acquiring the current operating data of the target atomizing sheet; calculating the driving parameters of the target atomizing sheet based on the atomizing sheet parameters and the current operating data to obtain the target driving signal; and driving the target atomizing sheet based on the current operating data and the target driving signal, the compatibility with various types of atomizing sheets is realized, and the real-time adjustment is achieved according to an operating state of the atomizing sheet, which improves the control efficiency and accuracy of the atomizing sheet.

The embodiments described in the embodiments of the present application are for the purpose of describing the technical solution of the embodiments of the present application more clearly, and do not constitute the limitation of the technical solution provided by the embodiments of the present application. Those skilled in the art know that, as the technical evolution and the emergence of new application scenarios, the technical solution provided by the embodiments of the present application is also applicable to similar technical problems.

Those skilled in the art can understand that the technical solution shown in the drawings does not constitute the limitation to the embodiments of the present application, and can include more or less steps than those shown in the drawing, or combine some steps, or different steps.

The device embodiment described above is only illustrative, where the units described as separate components may or may not be physically separated, which means that the units may be located in one place or distributed to multiple network units. Some or all of the modules may be selected according to actual needs to achieve the objects of the solutions in the embodiments.

Those of ordinary skills in the art can understand that all or some of the steps in the method, the system and the functional modules/units in the device disclosed above can be implemented as software, firmware, hardware and an appropriate combination thereof.

The terms "first", "second", "third", "fourth", and the like (if any) in the specification and the drawings of the present application above are used to distinguish similar objects, and are not necessarily used to describe a specific order or sequence. It should be understood that data used in this way can be interchanged under appropriate circumstances, so that the embodiments of the present application described herein can be implemented in a sequence other than those illustrated or described herein. In addition, the terms "including", "having" and any variations thereof are intended to cover non-exclusive inclusion. For example, a process, method, system, product or device including a series of steps or units is not necessarily limited to those steps or units clearly listed, but can include other steps or units not clearly listed in or inherent to the process, method, product or device.

It should be understood that, in the present application, "at least one (item)" refers to being one or more, and "multiple" refers to being two or more. "And/or" is used for describing the relationship between related objects, and indicates that there can be three relationships. For example, "A and/or B" can indicate that: A exists alone, B exists alone, and A and B exist at the same time, where A and B can be singular or plural. The symbol "/" generally indicates that there is a relationship of "or" between the related objects. "At least one (item) of the followings" or similar expression thereof refers to any combination of these items, including a singular (item) or any combination of plural (items). For example, at least one (item) of a, b or c can indicate: a, b, c, "a and b", "a and c", "b and c", or "a and b and c", where a, b and c can be singular or plural.

In the several embodiments provided in the present application, it should be understood that the disclosed apparatus and method can be implemented in other ways. For example, the foregoing device embodiments are only illustrative. For example, the division of the above units is only one logical function division. In practice, there can be other division methods. For example, multiple units or assemblies can be combined or integrated into another system, or some features can be ignored or not executed. In addition, the illustrated or discussed mutual coupling or direct coupling or communication connection can be indirect coupling or communication connection through some interfaces, devices or units, and can be in electrical, mechanical or other forms.

The units illustrated as separated parts above may be or may not be physically separated, and the parts displayed as units can be or not be physical units, which means that the parts can be located in one place or distributed on multiple network units. Some or all of the units can be selected according to actual needs to achieve the objects of the solutions of the embodiments.

In addition, each functional unit in each embodiment of the present application can be integrated in one processing unit, or each unit can exist alone physically, or two or more units can be integrated in one unit. The integrated units above can be implemented in a form of hardware, or can be implemented in a form of software functional unit.

The integrated units, if being implemented in the form of software functional unit and taken as an independent product to sell or use, can also be stored in one computer-readable storage medium. Based on such understanding, the essence of the technical solutions of the present application, or the part contributing to the prior art, or all or a part of the technical solutions can be embodied in the form of a software product. The computer software product is stored in a storage medium including a number of instructions such that a computer device (which can be a personal computer, a server, or a network device, etc.) performs all or a part of steps of the method described in each of the embodiments of the present application. The foregoing storage medium includes: any medium capable of storing programs such as a USB disk, a mobile hard disk, a Read-Only Memory (ROM), a Random Access Memory (RAM), a magnetic disk, an optical disk, or the like.

The preferred embodiments of the embodiments of the present application are described above with reference to the drawings, and are not intended to limit the scope of rights of the embodiments of the present application. Any modification, equivalent substitution and improvement made by those skilled in the art without departing from the scope and essence of the embodiments of the present application should be included within the scope of rights of the embodiments of the present application.

## Claims

1. A method for driving an atomizing sheet, comprising:
acquiring atomizing sheet parameters of a target atomizing sheet (S101);
acquiring current operating data of the target atomizing sheet (S102);
calculating driving parameters of the target atomizing sheet based on the atomizing sheet parameters and the current operating data to obtain a target driving signal (S103); and
driving the target atomizing sheet based on the current operating data and the target driving signal (S104).

2. The method according to claim 1, wherein the calculating the driving parameters of the target atomizing sheet based on the atomizing sheet parameters and the current operating data to obtain the target driving signal comprises:
determining reference operating data according to the atomizing sheet parameters (S201);
performing frequency sweeping on the target atomizing sheet based on the reference operating data and the current operating data to obtain an original driving frequency (S202); and
generating the target driving signal based on the original driving frequency (S203).

3. The method according to claim 2, wherein the current operating data comprises a current operating frequency and a current operating current, the reference operating data comprises a target operating frequency range, and the performing frequency sweeping on the target atomizing sheet based on the reference operating data and the current operating data to obtain the original driving frequency comprises:
acquiring a maximum value of the current operating current in a preset frequency sweeping period to obtain a maximum operating current (S301);
determining a maximum current operating moment according to the maximum operating current (S302);
acquiring a maximum current operating frequency from the current operating frequency based on the maximum current operating moment (S303); and
in response to the maximum current operating frequency being within the target operating frequency range, determining the maximum current operating frequency as the original driving frequency (S304).

4. The method according to claim 3, wherein the generating the target driving signal based on the original driving frequency comprises:
acquiring an original driving amplitude from the current operating data according to the original driving frequency (S501); and
modulating a driving signal based on the original driving frequency and the original driving amplitude to obtain the target driving signal (S502).

5. The method according to claim 3, wherein the current operating data further comprises current operating power and a current operating temperature, the reference operating data comprises preset minimum operating power and a preset low-temperature operating threshold, and the performing frequency sweeping on the target atomizing sheet based on the reference operating data and the current operating data to obtain the original driving frequency comprises:
in response to the current operating power being lower than the minimum operating power or the current operating temperature is lower than the low-temperature operating threshold, performing frequency sweeping on the target atomizing sheet based on a preset number of times of frequency sweeping to obtain a plurality of initial driving frequencies (S401); and
averaging the plurality of initial driving frequencies to obtain the original driving frequency (S402).

6. The method according to claim 5, wherein the reference operating data further comprises a preset hole plugging operating power range, and the driving the target atomizing sheet based on the current operating data and the target driving signal comprises:
in response to the current operating power being within the hole plugging operating power range, performing frequency conversion on the target driving signal based on the reference operating data to obtain a variable frequency driving signal (S601); and
driving the target atomizing sheet based on the variable frequency driving signal (S602).

7. The method according to claim 6, wherein the reference operating data further comprises a preset target operating power range, and after driving the target atomizing sheet based on the variable frequency driving signal, the method further comprises:
acquiring variable frequency operating power of the target atomizing sheet (S701); and
in response to the variable frequency operating power being within the target operating power range, driving the target atomizing sheet based on the target driving signal (S702).

8. An apparatus for driving an atomizing sheet, comprising:
an atomizing sheet parameter acquisition module (801) configured for acquiring atomizing sheet parameters of a target atomizing sheet;
a current operating data acquisition module (802) configured for acquiring current operating data of the target atomizing sheet;
a driving parameter calculation module (803) configured for calculating driving parameters of the target atomizing sheet based on the atomizing sheet parameters and the current operating data to obtain a target driving signal; and
an atomizing sheet driving module (804) configured for driving the target atomizing sheet based on the current operating data and the target driving signal.

9. An electronic device, wherein the electronic device comprises a memory (902) and a processor (901), the memory (902) stores a computer program, when executed by a processor (901), causes the processor (901) to perform the method for driving an atomizing sheet according to claim 1.

10. A computer-readable storage medium, wherein the computer-readable storage medium stores a computer program, that when executed by a processor (901), causes the processor (901) to perform the method for driving an atomizing sheet according to claim 1.
